# EUROPEAN PATENT SPECIFICATION

(11) **EP 4 093 333 B1**
(45) Date of publication and mention of the grant of the patent: **20.05.2026**
(21) Application number: 21744206.0
(22) Date of filing: 22.01.2021
(51) Int. Cl.: A61B 90/00, A61B 17/00, A61F 2/24, A61M 39/10, A61B 17/02

(54) **DEVICE FOR VISUALIZATION OF VALVE DURING SURGERY**
VORRICHTUNG ZUM SICHTBARMACHEN EINER HERZKLAPPE WÄHREND EINER OPERATION
DISPOSITIF DE VISUALISATION DE VALVE LORS D'UNE INTERVENTION CHIRURGICALE

(30) Priority: 24.01.2020 US 202062965726 P
(43) Date of publication of application: 30.11.2022
(73) Proprietor: Children's Medical Center Corporation, Boston, MA 02115 (US)
(72) Inventor: HOGANSON, David, Chestnut Hill, Massachusetts 02467 (US); HAMMER, Peter E., Needham, MA 02492 (US); BERRA, Ignacio, Boston, MA 02115 (US); DEL NIDO, Pedro J., Lexington, MA 02420 (US); PERRIN, Douglas P., Boston, MA 02115 (US)
(74) Representative: Hoffmann Eitle
(86) International application number: PCT/US2021/014798
(87) International publication number: WO 2021/151028

(56) References cited:
- EP-A1- 1 943 942
- WO-A1-2016/158294
- WO-A1-2018/062152
- WO-A1-96/32882
- US-A1- 2003 176 835
- US-A1- 2004 073 301
- US-A1- 2009 093 877
- US-A1- 2011 071 351
- US-A1- 2012 136 434
- US-A1- 2020 060 819
- US-A1- 2020 060 819

## Description

### FIELD

The disclosed embodiments relate to the repair and/or replacement of heart valves by utilizing a device to visualize a heart valve during heart valve repair or replacement.

### BACKGROUND

Surgical repair or replacement of the aortic or pulmonary valve is a common procedure for both children and adults. For example, the valves may become narrowed or leaky, which may necessitate an intervention, such as a surgical intervention. Typically, after a repair or replacement of the aortic or pulmonary valve, the heart and great vessels are closed, the patient is weaned off of bypass (e.g., the heart/lung machine), and the repair or replaced valve(s) is assessed via an echocardiogram to understand the function of the valve(s) after the intervention. If there is a problem with a valve after the intervention, e.g., the valve is not functioning or there is a leak around the interface between the edge of the valve and annulus of the heart where the valve should be opposed, further surgical intervention may be needed, which may require the patient to again be placed on bypass.

US 2011/0071351 A1 discloses a device for evaluating an aortic valve after opening an aorta distal to the aortic valve. The device includes a body defining a chamber. The body has a generally circular proximal open end and a distal end, the proximal open end being configured to be reversibly attachable to one of the aorta and a short open tube distal to the aortic valve in a generally water-tight fashion. At least a portion of the body is transparent to view the aortic valve.

WO 2018/062152 A1 discloses a medical leak inspection device.

WO 2016/158294 A1 discloses a medical leak inspection device.

EP 1943942 A1 discloses an examining device for examining an aortic valve and a pulmonary valve competency.

### SUMMARY

According to the present invention, there is provided a viewer according to claim 1.

The dependent claims define preferred embodiments.

A method of viewing one or more valve leaflets via a viewer having a body with a first end and a second end opposite the first end is disclosed but not encompassed by the scope of the claims. The method includes attaching the second end of the viewer to an aorta or a pulmonary artery, pressurizing the valve leaflets via one or more ports attached to the body, and viewing the one or more valve leaflets via a transparent viewing window of the first end of the body, wherein an outer diameter of the first end of the body is larger than an outer diameter of the second end of the body and/or wherein a diameter of the viewing window is larger than the outer diameter of the second end of the body.

The foregoing and other aspects, embodiments, and features of the present teachings can be more fully understood from the following description in conjunction with the accompanying drawings.

### BRIEF DESCRIPTION OF THE DRAWINGS

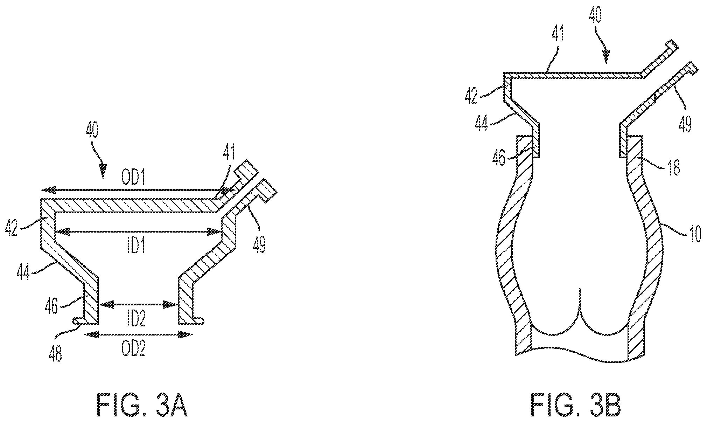

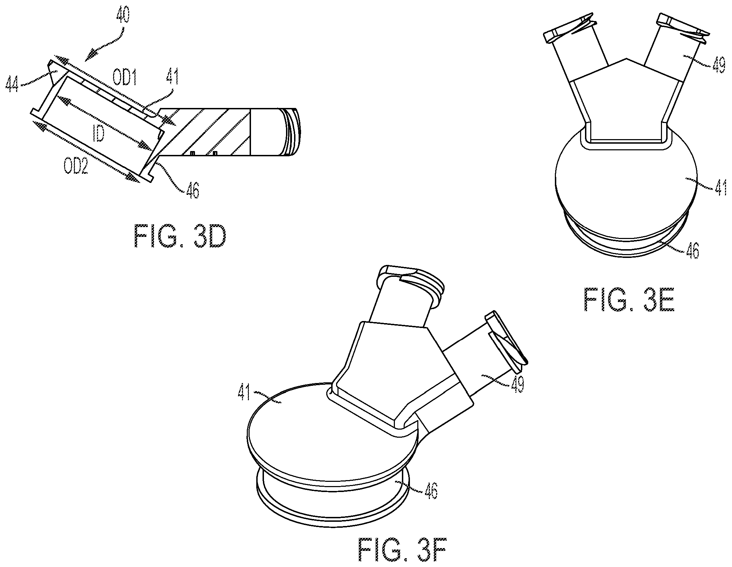

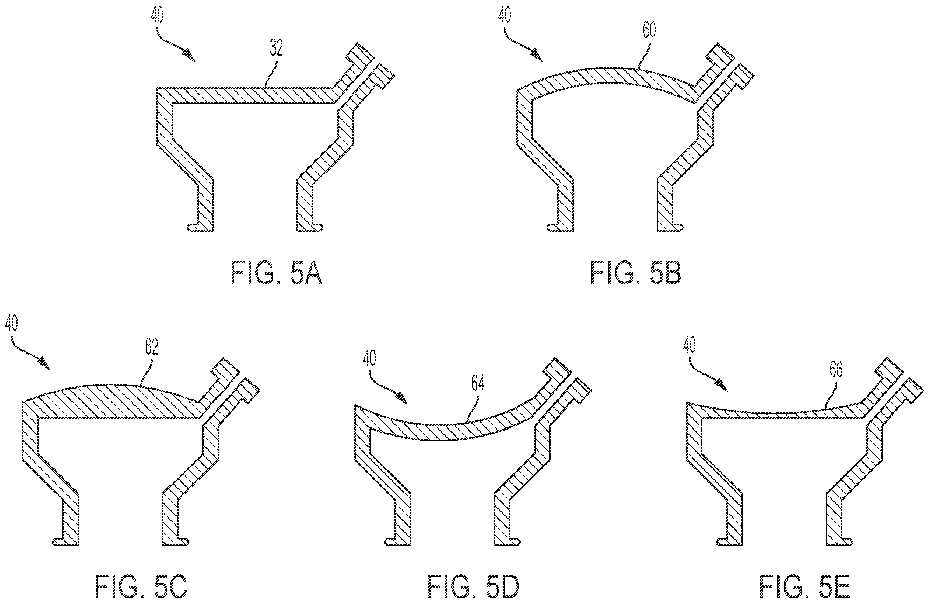

The accompanying drawings are not intended to be drawn to scale. In the drawings, each identical or nearly identical component that is illustrated in various figures is represented by a like numeral. Figures 3A and 5A-E show a viewer in accordance with the invention. For purposes of clarity, not every component may be labeled in every drawing. In the drawings:
FIG. 1A is a cross-sectional view of the ascending aorta looking down into the aortic root and visualization of the aortic valve;
FIG. 1B is a longitudinal cross-sectional view of an aortic root and ascending aorta into aortic valve leaflets;
FIG. 1C is a cross-sectional view of the ascending aorta looking down into the aortic root with visualization of the aortic valve leaflets and a central area of non-coaptation;
FIG. 1D is a cross-sectional view of the ascending aorta looking down into the aortic root with visualization of the aortic valve leaflets and an eccentric area with inadequate coaptation;
FIG. 1E is a longitudinal cross-sectional view of the aortic root in the ascending aorta in an unpressurized state with visualization of two aortic valve leaflets;
FIG. 1F is a longitudinal cross-sectional view of the aortic root and the ascending aorta in a pressurized state with visualization of two aortic valve leaflets;
FIG. 2A is a longitudinal cross-sectional view of an aortic root and an ascending aorta with a view of two aortic valve leaflets after repair in an unpressurized aortic root;
FIG. 2B is a cross-sectional view of a device for viewing the aortic valve, not encompassed by the scope of the claims;
FIG. 2C is a longitudinal cross-sectional view of the aortic root and ascending aorta with a valve viewing device, not encompassed by the scope of the claims, placed in the ascending aorta for viewing the aortic valve with the aortic root pressurized;
FIG. 2D is an isometric view of an aortic valve viewing device, not encompassed by the scope of the claims;
FIG. 2E is an isometric view of the device of FIG. 2D positioned within the ascending aorta;
FIG. 3A is a cross-sectional view of a valve viewing device according to the invention;
FIG. 3B is a cross-sectional view of a valve viewing device positioned within the ascending aorta;
FIG. 3C is an isometric view of a valve viewing device positioned within the ascending aorta;
FIG. 3D is a cross-sectional view of another valve viewing device;
FIG. 3E is a top perspective view of the valve viewing device of FIG. 3D;
FIG. 3F is another perspective view of the valve viewing device of FIG. 3D;
FIG. 4A is a cross-sectional view of a valve viewing device positioned within the ascending aorta with visualization of the aortic root and filling of the aortic root with a fluid;
FIG. 4B is a longitudinal view of a valve viewing device positioned within the ascending aorta with visualization of the aortic root and coronary arteries with temporary occlusion of one or more coronary arteries;
FIG. 5A is a cross-sectional view of a valve viewing device, according to the invention, with a viewing window that is flat;
FIG. 5B is a cross-sectional view of a valve viewing device, according to the invention, with a viewing window that is convex;
FIG. 5C is s cross-sectional view of a valve viewing device, according to the invention, with a portion of the viewing window that is convex;
FIG. 5D is a cross-sectional view of a valve viewing device, according to the invention, where the viewing window that is concave;
FIG. 5E is a cross-sectional view of a valve viewing device, according to the invention, where at least a portion of the viewing window is concave;
FIG. 6A is an isometric view a valve viewing device demonstrating the radial position of two ports on the device;
FIG. 6B is an isometric view of a valve viewing device demonstrating the radial position of two ports on the device;
FIG. 6C is an isometric view of a valve viewing device demonstrating the position of two ports on the device;
FIG. 6D is a partial cross-sectional view of the valve viewing device of FIG. 6C;
FIG. 6E is an isometric view of a valve viewing device with a bifurcated port configuration;
FIG. 6F is a modified sectional view of the valve viewing device of FIG. 6E;
FIG. 7A is a cross-sectional view of a valve viewing device, not encompassed by the scope of the claims;
FIG. 7B is a cross-sectional view of a valve viewing, not encompassed by the scope of the claims;
FIG. 7C is a cross-sectional view of a valve viewing device not encompassed by the scope of the claims;
FIG. 7D is a cross-sectional view of a valve viewing device not encompassed by the scope of the claims;
FIG. 8A is a cross-sectional view of a valve viewing not encompassed by the scope of the claims;
FIG. 8B is a cross-sectional view of a valve viewing device not encompassed by the scope of the claims;
FIG. 8C is a cross-sectional view of a valve viewing device not encompassed by the scope of the claims;
FIG. 8D is a partial sectional view of a valve viewing device according to another embodiment;
FIG. 9A is a longitudinal cross-sectional view of an ascending aorta and aortic root with demonstration of the sinotubular junction;
FIG. 9B is a cross-sectional view of a valve viewing device situated within an ascending aorta;
FIG. 9C is a cross-sectional view of a valve viewing device situated within an ascending aorta that is smaller than near the base aorta modifying the sinotubular junction;
FIG. 9D is a cross-sectional view of a valve viewing device situated within an ascending aorta where the device is larger in diameter than the ascending aorta and impacts the sinotubular junction dimension of the aorta;
FIG. 10A is a cross-sectional view of a valve viewing device positioned within the ascending aorta and reversibly secured to the ascending aorta;
FIG. 10B is a side view of a valve viewing device within the ascending aorta with an aortic valve viewing device reversibly secured to the ascending aorta;
FIG. 10C is a cross-sectional view of a valve viewing device positioned within the ascending aorta and reversibly secured to the ascending aorta;
FIG. 10D is a cross-sectional view of a valve viewing device positioned within the ascending aorta and reversibly secured to the ascending aorta;
FIG. 10E is a side view of a valve viewing device within the ascending aorta with the device reversibly secured to the ascending aorta;
FIG. 10F is a cross sectional view of portion of a valve viewing device not encompassed by the scope of the claims, and a securement ring;
FIG. 10G is a cross sectional view of the valve viewing device of FIG. 10F with the securement ring in place to reversibly secure the device to the ascending aorta;
FIG. 10H is a cross-sectional view of a valve viewing device, not encompassed by the scope of the claims, positioned within the ascending aorta with a securement band in position to reversibly secure the ascending aorta to the aortic valve viewing device;
FIG. 11A is a cross-sectional view of a multi-component valve viewing device, not encompassed by the scope of the claims, reversibly secured to the ascending aorta;
FIG. 11B is a cross-sectional view of another multi-component valve viewing device, not encompassed by the scope of the claims, reversibly secured to the ascending aorta;
FIG. 12A is a cross-sectional view of a valve viewing device, not encompassed by the scope of the claims, with a scope interfacing;
FIG. 12B is a cross-sectional view of a valve viewing device, not encompassed by the scope of the claims, with a scope interfacing;
FIG. 12C is a cross-sectional view of a valve viewing device, not encompassed by the scope of the claims, not encompassed by the scope of the claims with a scope interfacing;
FIG. 12D is a cross-sectional view of a multi component valve viewing device, not encompassed by the scope of the claims, and a scope interfacing with the device;
FIG. 12E is a cross-sectional view of a multi component valve viewing device, not encompassed by the scope of the claims, with a scope positioned within the device seal;
FIG. 13A is a cross-sectional view of a valve viewing device with multiple ports;
FIG. 13B is a cross-sectional view of a valve viewing device, not encompassed by the scope of the claims, with multiple ports with luer connections;
FIG. 13C is a cross-sectional view of a valve viewing device, not encompassed by the scope of the claims, with multiple ports and luer lock connectors;
FIG. 13D is an isometric view of a valve viewing device, not encompassed by the scope of the claims, with multiple ports and luer lock connectors;
FIG. 13E is a cross-sectional view of a valve viewing device, not encompassed by the scope of the claims, within the ascending aorta where the ascending aorta is cross clamped and pressurized, and a scope is positioned within the aortic valve viewing device to visualize the aortic valve; and
FIG. 13F is a cross-sectional view of a valve viewing device, not encompassed by the scope of the claims, positioned within the ascending aorta where the ascending aorta is crossed clamped and the scope is positioned within the aortic valve viewing device for visualization of the aortic valve.

### DETAILED DESCRIPTION

Aortic and pulmonary valves are tricuspid valves designed to open during a systolic phase of ventricular contraction to conduct blood to the aorta and pulmonary artery and then close completely during diastole to prevent backflow of the blood into the left ventricle or right ventricle.

The pulmonary and aortic valves often require intervention in children and adults due to congenital and/or acquired diseases. The aortic valve requires intervention much more commonly in adults than in children. Typically, intervention on the aortic valve may include either repair of the valve and/or replacement of the valve, such as with a bioprosthetic or a mechanical valve. Valve repair techniques exist for both children and adult valves and may range from placement of a few sutures to support the valve to replacement of one or more leaflets of the valve with prosthetic leaflet material. Repair and/or replacement of the pulmonary or aortic valve is commonly done with the patients being supported by bypass (i.e., via a heart/lung machine), with the heart stopped, and with the surgeon working on the heart while it is still and the chamber below the valve is depressurized. As will be appreciated, this creates an ideal environment that may be cleared of blood without significant motion and allow for a meticulous repair or replacement heart valve.

Aortic or pulmonary valve repairs can be quite complex in nature. This can involve repairing one or each of the leaflets of the aortic or pulmonary valves. At the simplest end, some stitches to support the valve or shape the aortic valve annulus can be required. There can be a deficiency of leaflet material requiring enlargement or elongation of the leaflets with a patch material. There can be one small or deficient leaflet that is cut out entirely, as in the situation of a quadricuspid aortic valve. This can require a replacement of one, two, or all three leaflets with prosthetic material. A technique to replace all three leaflets with leaflets constructed of a different material has emerged as a very useful technique called the Ozaki repair. In this technique all three leaflets are placed either with autologous pericardium or bovine or equine pericardial material. With both aortic valve repair and replacement techniques, there is need to understand with great detail the coaptation of the leaflets, i.e., the overlap of two adjacent leaflets that forms a seal for the valve.

The inventors have recognized that assessment of an intervention presents a challenge to intervening on the aortic or pulmonary valve. For example, typically, after a repair or replacement of the aortic or pulmonary valve, the heart and great vessels are closed, the patient is weaned off of bypass, and the repair or replaced valve(s) is assess via an echocardiogram to understand the function of the valve after the intervention. If there is a problem with the valve(s) after the intervention, e.g., the valve is not functioning or there is a leak around the interface between the edge of the valve and annulus of the heart where the valve should be opposed, additional surgical intervention(s) may be needed, which may require the patient to again be placed on bypass. Such a return to bypass, which may include one or more additional times on the heart/lung machine to reintervene on the valve, may add time, expense, and risk to the procedure and to the patient. Additionally, the clarity of information that is available from an echocardiogram is limited in nature. Accordingly, the inventors have recognized that benefits may be realized by assessing the intervention immediately after it was completed to understand the effect of that intervention. For example, benefits may be realized by observing a repaired or replaced heart valve by direct vision with the heart still opened.

In some instances, visualization may be done concurrently with the intervention, however, in such instances, the valve is visualized with the aorta still opened such that there is zero pressure on the valve. The heart surgeon may squirt a little saline on top of the valve to make sure that the leaflets come together, but this nearly zero-pressure assessment of the valve function is a poor approximation of how the valve would perform once the great vessel is closed and the valve is working under normal physiologic conditions. Accordingly, the inventors have recognized that benefits may be realized by visualization of the valve while the aortic root or the pulmonary root is pressurized to a pressure in the same range of normal or exceptional physiologic conditions. For example, pressurization of the aortic root with direct visualization could allow for detailed examination of the individual leaflets and how they push together or coapt to form a seal for the valves. In such an example, this may provide a truer assessment of the heart valve function and give the surgeon confidence that the valve may work well when the patient is off bypass and when the patient's blood pressure is within the normal range. This also could allow for precise revision of any repair and for development of new techniques or modifications of repair approaches that could be learned from detailed visual inspection of aortic or pulmonary valves after repair. In some embodiments, visualization may be performed when the aortic root or the pulmonary root is pressurized at elevated pressures. For example, as an individual's blood pressure may rise substantially during exercise, visualization over a range or normal and elevated pressures may provide the surgeon with an assessment of the how the heart valve may function under different conditions.

The present disclosure describes the repair and/or replacement of heart valves and utilization of an imaging device, also referred to herein as a visualization device, a viewing device, an imager, a viewer, and/or the device, to evaluate heart valve function of the pulmonary and/or aortic valve by direct visualization.

The viewer includes a body having a first end through which a surgeon may view and evaluate heart valve function of the pulmonary or aortic valve and a second end arranged to be attached to the aorta. The first end includes a transparent viewing window through which the surgeon may visualize the pulmonary and/or aortic valve.

In some embodiments, the body includes a tubular body. In some embodiments, the tubular body may have a diameter that is the same between the first, viewing end and the second, attachment end. For example, in some embodiments, the outer diameter of the tubular body may be the same from the first end to the second end. In other embodiments, the diameter between the first and second ends may vary. For example, in some embodiments, the tubular body may have a diameter that decreases between the viewing end and the attachment end. For example, in some embodiments, the outer diameter of the tubular body may be larger at the first, viewing end than at the second, attachment end. In some embodiment, the tubular body may decrease in a stepped manner, with a first body portion having a first diameter and a second body portion having a second diameter that is different than the first diameter. The body also may include one or more tapered portions. In some embodiments, the first, viewing end may be closed and the second, attachment end may be open.

In some embodiments, the inner and outer diameters of the first, viewing end of the body may be larger than the respective inner and outer diameters of the second, attachment end. In other embodiments, the inner diameter of the body may be the same between the first and second ends, although the outer dimeter of the first end is greater than the outer diameter of the second end. In some embodiments, the viewer is arranged such that the viewing window has a diameter that is larger than an outer diameter of the second end of tubular body. In such embodiments, the viewing window may have a diameter that is also larger than the inner dinner of the tubular body. In some embodiments, the viewing window may be the same diameter as the first end of the tubular body, although the viewing window also may have a diameter that is smaller than the diameter of the first end of the tubular body.

In some embodiments, the viewer may allow for pressurization of the aortic or pulmonary root with a fluid and visualization of the function of the pulmonary or aortic valve under pressure. For example, in some embodiments, the device may have the capacity to both fill the root with a fluid, for example an electrolyte solution which is optically clear (e.g., as plasmalyte), and also de-air the root to allow an unobstructed view of the aortic or pulmonary valves under pressure. The device includes one or more ports to allow for providing a fluid to the root and/or to de-air the root. For example, in some embodiments, the device may include a first, fluid supply port, and a second, fluid return or outlet port (e.g., for evacuation of air). In some embodiments, the ports may be used to connect respective inlet and outlet tubing to the device. In other embodiments, a single port may include first and second fluid lines. For example, a single port may include a first fluid line to provide fluid to the device and a second fluid line to evacuate fluid (e.g., air). The ports are be arranged to provide fluid to and/or evacuate fluid from an internal portion of the viewer.

In some embodiments, the ports may be positioned on a side of the viewer so as to not obstruct visualization through the viewing window of the device. For example, in some embodiments, the ports may be attached at a periphery of the first end of the viewer, such as where a top of the viewer body joins the side of the body. As will be appreciated, the ports also may be located on another suitable portion of the device, such as on the viewer window itself. In some embodiments, the ports may be attached at an angle relative to the body, and to a plane of the viewing window. The ports also may be attached parallel and/or perpendicular to a plane of the viewing widow.

In some embodiments, utilizing the device, the aortic or pulmonary valves can be visualized at an angle such that a valve may be viewed in as undisturbed of a native position as possible. In some embodiments, the device may allow for utilization of a camera-based imaging system with a device for recording or projection of the view of the valve on a screen or to a computer or other recording device.

In some embodiments, the viewer may allow for magnification of the aortic valve leaflets through a lens or broadening the view of the aortic valve root through a lens technology incorporated within the aortic valve viewer. In some embodiments, magnification or wide-angle visualization of a portion of the aortic valve may be performed via the viewing device. In other embodiments, such magnification and/or wide-angle visualization may be a feature of the visualization camera or optical system that interfaces directly with the viewer.

In some embodiments, the viewer may be reversibly secured within the ascending aorta, main pulmonary artery, or graft replacement of one of these vessels. For example, the device may be reversibly secured to the ascending aorta or aortic root utilizing a suture, cable, or other filament-like structure around the aortic root. Alternatively, other mechanical means including elastic bands, clip-like features, or a collet or clamp could be used to secure the aortic root or ascending aorta to the device. In another embodiment threaded or screw-like feature could be used to anchor multiple parts of the valve viewing device together to secure it to the aorta. As will be appreciated, more than one type of fastening or securement features may be used to secure the viewer to the ascending aorta.

In some embodiments, the viewer may be secured after a surgical intervention, the valve(s) may be visualized under pressure, and the viewer then may be removed. In some embodiments, after the viewer is removed, an additional intervention may be conducted, and the viewer may be attached a second time. In other embodiments, after the viewer is removed, the surgical field may be closed, and the patient weaned off of bypass.

In some embodiments, the device may be advanced through an opening in the pulmonary valve or aortic valve root or ascending aorta or main pulmonary artery to provide visualization of the valve and pressurization of that vessel. This may include a visualization system such as a laparoscope or other straight, angled, or adjustable device to gain adequate visualization of the valve.

In some embodiments, the device may include one or more openings through which one or more surgical tools may be inserted. In such embodiments, a surgeon may perform a surgical step, or a part of another intervention, without having to first remove the viewer. A surgeon also may insert an imaging device through the one or more openings of the viewer.

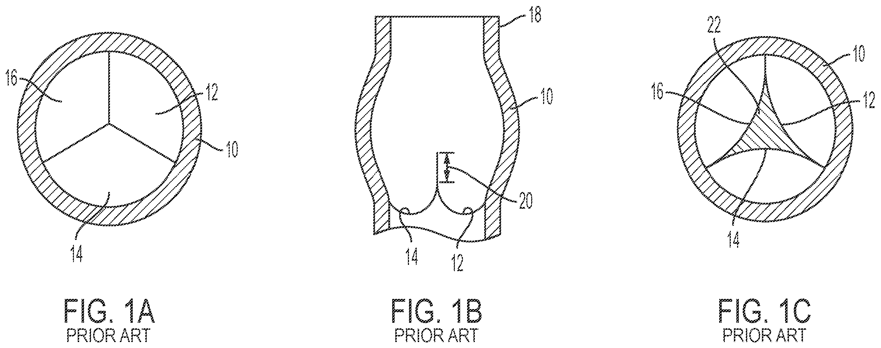

Turning now to the figures, FIG. 1A shows a cross section of the aorta 10 viewed from a position in the ascending aorta looking down into the aortic valve. As shown in this view, the aortic valve includes three aortic valve leaflets 12, 14, and 16, which are in a closed position in this view. As is known, the aortic valve and pulmonary valve leaflets achieve closure by pushing up against each other over a distance called the cooptation height. In normal aortic and pulmonary valves, the leaflets are at the same height relative to each other. When the aortic valve is viewed from above the valve, one can see the valve leaflets come together to form a seal when the valve root is pressurized.

Referring to FIG. 1B, a longitudinal cross-sectional view of the aortic root 10 and ascending aorta 18 is shown with a view of two aortic valves 12 and 14 which are coapting together. A coaptation height 20 is demonstrated as a height over which the two leaflets are pressed together to form a seal during diastole. As will be appreciated, the coaptation height in a normal valve varies according to the size of the patient and aortic valve annulus. As is known, the coaptation height is a central measurement of the function of the valve and the margin of the valve to remain competent over a range of physiologic conditions.

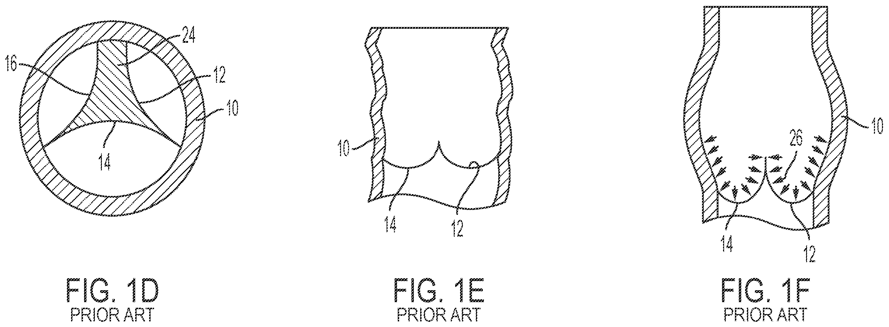

FIG. 1C shows a cross-section of the ascending aorta 10 with aortic valve leaflets 12, 14, and 16 that do not completely come together and with a central area 22 between the leaflets. In this instance, there is an area of non-coaptation in the central portion of the valve. As will be appreciated, for an aortic valve that is dysfunctional, a common location of the valve leak is in the center of the valve between the three leaflets. Other versions of aortic or pulmonary valves may have two leaflets, known as a bicuspid or bi-leaflet valve, or four or more leaflets, known as quadricuspid valve, or other variations of leaflets as part of congenital heart disease. Areas where the leaflets do not completely come together during diastole when the valve is loaded with pressure may result in reversal of flow through the valve or regurgitation. Referring to FIG. 1D, the aortic root 10 and aortic valve leaflets 12, 14, and 16 are shown in cross section with an eccentric area 24 where there is inadequate coaptation between the aortic valve leaflets 12 and 16. As will be appreciated, areas of eccentric non-coaptation between two adjacent leaflets may result in eccentric regurgitation through the valve and may require intervention on one or more leaflets to correct.

The appearance and function of the aortic valve varies depending on the pressures in the aortic root and ascending aorta. Referring to FIG. 1E, the aortic root 10 is shown in a non-pressurized configuration, which demonstrates leaflets 12 and 14 that are opposed but barely so, and with a very short coaptation height. Referring to FIG. 1F, when the aortic root 10 is pressurized, the pressure on the aortic valve leaflets (see the arrows labeled 26) may impart a load on the leaflets which may increase the height and area over which the valves coapt. This, in turn, may increase the coaptation height and change the appearance of the leaflets substantially. Accordingly, a pressurized or loaded aortic root and leaflet may give the surgeon a more accurate assessment of the function of the aortic valve when directly inspected.

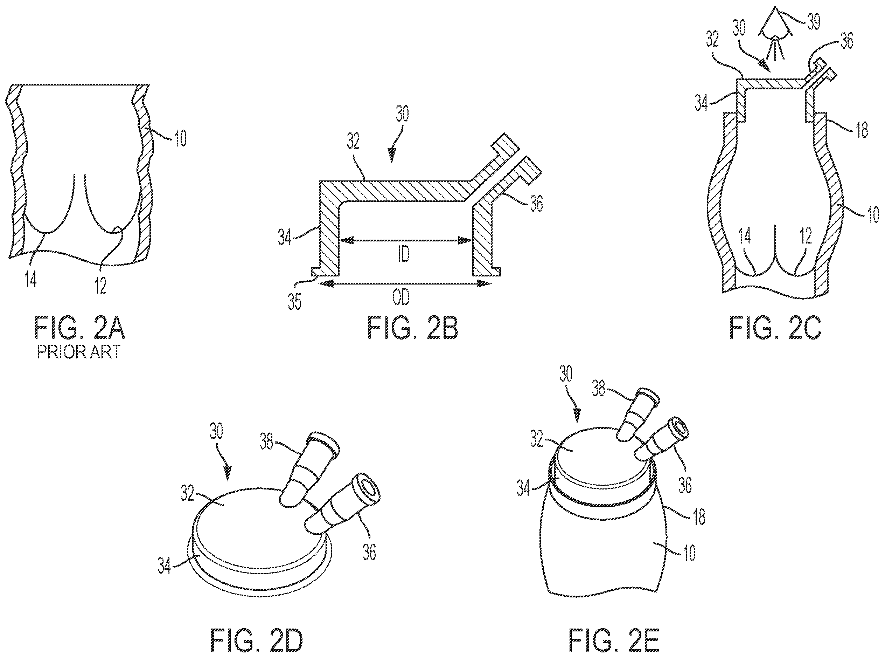

Referring to FIG. 2A, the aortic root 10 is shown in a longitudinal cross section in an unpressurized state where the aortic valve leaflets 12 and 14 are shown in a near coaptation state. As will be appreciated, this is commonly what the aortic valve may look like after repair when the ascending aorta is opened and non-pressurized. In such instances, the surgeon may pull the leaflets together with forceps to try to assess the relative leaflets heights, but it is impossible to know exactly what the aortic valve will look like when it is pressurized or loaded and the leaflets are pushed together.

FIG. 2B shows a valve viewing device 30 according to embodiments of the present disclosure. As shown in this cross-sectional view, the device includes a body 34 with a viewing window 32 arranged to allow a surgeon to view one or more valves. The viewing window is transparent. For example, the viewing window may include an optically clear surface through which the surgeon may view the valves.

As will be appreciated, the viewing device may be used on either the aorta or pulmonary valve, even though the device itself may be referred to as an aortic or pulmonary valve device, respectively. As will be appreciated, although the viewing devices is described for use to view valves, the viewing device may be used in other suitable portions of the body.

The viewing window may incorporate curvature on one or more than one surfaces to optimize the optical performance of the device to reduce glare, reflection or distortion. Coatings may be added to one or more surface to reduce glare, reflection, distortion, condensation or fogging. The coating may reduce surface tension of a liquid in contact with the coating. Likewise, in another embodiment, the thickness of the viewing window may vary to optimize optical performance including the reduction of glare, reflection or distortion. A polarizing filter, coating, lens or other technology that achieves polarization of light may be incorporated into or adjacent to the viewing window in some embodiments.

As shown in FIG. 2B, the viewing window may be located on a top of the body. For example, the viewing window may be located at a first end of the body, with the second, opposite end of the body being arranged to interface with the ascending aorta. As shown in FIG. 2B, the top of the body may be closed, while the second end of the body.

The viewing device includes one or more securement features, also referred to as attachment features, to assist in securing the device to the aorta or pulmonary artery. For example, the securement features may allow the device to be removably secured to the aorta or pulmonary artery. In some embodiments, a securement feature 35 (see FIG. 2B) may be positioned along a portion of body of the device to assist in reversibly securing the device to the aorta or pulmonary artery. In some embodiments, as shown in FIG. 2B, the securement feature may be located at or near the second, attachment end of the viewer. As will be appreciated, the securement feature(s) may be located around at least a portion of a circumference of the viewer. In some embodiments, the securement feature(s) may be located around an entire circumference of the viewer. In some embodiments, the securement features may be located near the second end of the viewer, although they also may be located at a central portion of the body or even at the first end of the body.

In some embodiments, the securement feature may include a mechanical means such as one or more barbs, elastic bands, clip-like features, or a collet or clamp that may be used to secure the aortic root or ascending aorta to the device. In another embodiment threaded or screw-like feature could be used to anchor multiple parts of the valve viewing device together to secure it to the aorta.

In some embodiments, the device may be reversibly secured to the ascending aorta or aortic root utilizing a suture, cable, or other filament-like structure around the aortic root. In accordance with the invention, the securement feature includes a rim extending outwardly from the second, attachment, end of the device, with the suture, cable or other filament-like structure being attachable around the aortic root, above the securement feature.

As will be appreciated, the device may include more than one of the same securement feature, or may use more than one type of securement feature. For example, the device may include one or more barbs and may be secured via a suture in some embodiments.

FIG. 2C illustrates securement of the valve viewing device 30, e.g., via the second end of the body 34, to the ascending aorta 18, which is continuous with the aortic root 10. As shown in this view, the first, viewing end of the body and the viewing window 32 are positioned opposite to the second, attachment end of the device and to the aortic valve leaflets 12 and 14. As shown in FIGS. 2B and 2C, the attachment end of the device may be open while the top end (e.g., the viewing end is closed).

In some embodiments, as shown in FIGS. 2B and 2C, the viewer 30 may include one or more ports (e.g., port 36), arranged to provide fluid access to and from an internal portion of the viewing device. For example, there may be one, two or more ports positioned to provide fluid access to the viewing device. In some embodiments, the port(s) may provide fluid access to and form an internal portion of the body. In some embodiments, the viewer also may include one or more ports arranged to allow instrument access within the aortic valve viewer device 30.

As shown in FIG. 2C, the aortic root and ascending aorta below the viewing device may be pressurized by insertion of fluid through the port 36 of the device and into the internal portion of the device. A surgeon may then be able to visualize the valve leaflets under pressure. For example, as shown in FIG. 2C, a representation of a surgeon's eye 39 is shown above the window 32 to illustrates visualization of the aortic valve leaflets 12 and 14 via the window 32 to assess the position of those leaflets and the expected function of the aortic valve in a pressurized state.

In some embodiments, as shown in FIGS. 2D and 2E, isometric views of the valve viewer 30, the viewer may include ports 36 and 38 which provide lumens for fluid communication with the internal portion of the viewing device. In one embodiment, ports 36 and 38 provide access for infusion of fluid through one port (e.g., port 36) and removal of air and excess fluid at the second, separate port (e.g., port 38). FIG. 2E illustrates use of one embodiment of the device and positioning of the device within the ascending aorta (e.g., in a reversible way) to provide fluid into the aorta via port 36 and as well as at removal of air or fluid through port 38.

As shown in FIG. 2B, in some embodiments, the body of the viewer may include a tubular body. As shown in this view, the body may include an outer diameter ***OD*** which is uniform throughout the body. For example, the outer diameter of the body may be the same at the first and second ends, and in between the first and second ends of the body. As also shown in FIG. 2B, in some embodiments, the tubular body may have an inner diameter ***ID*** that is uniform throughout the body (e.g., between the first and second ends of the body).

In accordance with the invention, the diameter of the body varies between the first and second ends of the viewer. As shown in FIG. 3A, the valve viewer 40 has a body with an outer dimeter ***OD1*** that is larger at the first, viewing end that the outer diameter ***OD2*** at the second, attachment end. In some embodiments, as shown in FIG. 3A, the inner diameter ***ID1*** of the first end may be larger than the inner diameter ***ID2*** at the second end. In some embodiments, as shown in FIG. 3D, the outer diameter of the body may vary between the first and second ends (see diameters ***OD1*** and ***OD2***), while the inner diameter ***ID*** of the body is the same between the first and second ends.

As shown in FIG. 3A, the body of the viewer includes a first portion with a first outer diameter ***OD1*** (see the portion of the body of the device 42 below the viewing window 41), and a second portion (see the portion of the body labeled 46) with a second outer diameter ***OD2.*** As shown in this view, the first diameter is larger than the second diameter. As also shown in FIG. 3A, the body of the viewer may have a tapered portion 44 where the diameter decreases from the first diameter to the second diameter. As shown in FIG. 3A, this angled, tapered portion of the body may connect the larger diameter portion of the body 42 and the smaller diameter portion of the body 46. In such embodiments, the outer diameter of the tapered portion decreases from the first outer diameter ***OD1*** to the second outer diameter ***OD2.***

As also shown in FIG. 3A, the first portion of the body 42 may include a first inner diameter, while the second portion of the body 46 includes a second inner diameter ***ID1**.* The tapered portion of the body 44 may include an inner diameter that tapers from the first inner diameter ***ID1*** to the second inner diameter ***ID2**.*

As will be appreciated, although the body is shown as having two stepped portions, portion 42 with the larger inner and outer diameters and portion 46 with the smaller inner and outer diameters, the body also may have more than two stepped portions with corresponding diameters in some embodiments. The body also may include only a single stepped portion and a single tapered portion. In other embodiments, the body also may include more than one tapered portion. For example, the body may include three stepped portions and two tapered portions in some embodiments. In still other embodiments, the body may have no stepped portions but may instead have only a single tapered portion, with the diameter of the body decreasing from the larger diameter at the first viewing end to the smaller diameter at the second, attachment, end.

FIG. 3D illustrates an embodiment in which the outer diameter of the viewer varies between the first and second ends, while the inner diameter remains constant. As shown in this view, the outer diameter ***OD1*** of the first end is larger than the outer diameter ***OD2*** of the second end of the body. In this embodiment, the outer diameter includes a tapered portion 44 below the viewing window 41, which decreases from the first outer diameter OD1 to the second outer diameter **OD2.** In this embodiment, the inner diameter ***ID*** is constant between the first and second ends of the body.

In some embodiments, the outer diameter of the second end may be between about 9mm and about 35 mm. As will be appreciated, the second, attachment end may be sized to match the size of the aorta of different patients. In instances where the device is sized for pediatric patients, the outer diameter of the second, attachment end may be between about 9 mm and about 19 mm. For example, the device may be 9 mm, 11 mm, 13 mm, 15 mm, or 19 mm in diameter for pediatric patients. In some embodiments, the device may be formed at different diameters, with a surgeon being able to select the desired sized viewer to attach to the patient during the intervention.

In some embodiments, the outer diameter of the first end may be between 2 mm and about 10 mm larger than the outer diameter of the second end. In some embodiments, a diameter of the first end may be two times larger than a diameter of the second end.

In some embodiments, the viewing widow may have a dimeter that is the same as that of the outer diameter of the first end of the body. As will be appreciated, in such embodiments, the first end of the body may be entirely formed by the viewing window. In other embodiments, the diameter of the viewing window may be the same size as the outer diameter of the second, attachment end of the body, although the outer diameter of the first end of the body may be greater than the outer diameter of the second end of the body. In such embodiments, a flange may extend axially around the viewing window, such as to attach the viewer window to the body. In other embodiments, the diameter of the viewing window may be larger than the outer diameter of the second, attachment end, while still being smaller than the outer diameter of the first, viewing end.

FIGS. 3A and 3D show embodiments where the diameter of the viewing window 41 may be larger than the outer diameter of the body at the second end, the inferior portion 46 of the valve viewer. In such embodiments, the diameter of the viewer window may be the same as the outer diameter of first end or may differ. For example, in one example, the viewer window may have a diameter that is larger than that of the second end of the body but is smaller than the first end of the body.

In some embodiments, a viewing window 41 with a larger diameter than the outer diameter of the second attachment end of the body, e.g., the portion of the body 46 that interfaces with the ascending aorta, may allow for a straight or oblique view of the aortic valve. In such embodiments, the viewer need not be perfectly in line with the vale to see the boundaries of the valve. In some embodiments, a viewing window with a larger diameter than the outer diameter of the second attachment end of the body also may assist in maintaining a position of the viewer in the aorta during the intervention.

In some embodiments, the relative diameters of the viewer window and the lower portion of the aortic valve viewer may range from being the same size or nearly the same size to the larger diameter of the viewing window being two times or larger than that of the lower portion of the body of the aortic valve viewer (e.g., the outer diameter of the second end of the body).

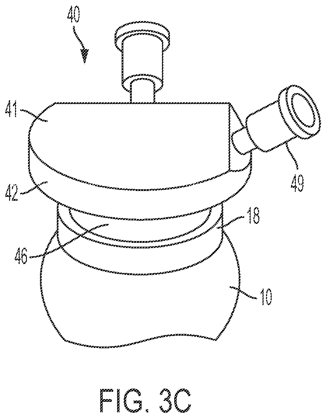

FIG. 3B shows the viewer 40 with the viewer window 41 being larger 42 than the portion of the viewer 46 engaging with ascending aorta 18 which is contiguous with the aortic root 10. As also shown in this embodiment, the viewer may include a port 49, such as to introduce fluid through the aortic valve viewer and pressurize the aortic root to allow the aortic valve function to be assessed by looking through the aortic valve viewing window 41. FIG. 3C shows an isometric view of the viewer interfaced at the lower portion of the body 46 with ascending aorta 18 contiguous with the aortic root 10. As will be appreciated by FIGS. 3B and 3C, the viewing window diameter, as demonstrated by the body of the upper body portion 42, may be larger than the ascending aorta 18 diameter. Such a viewer may allow a wider viewing angle into the aortic valve.

In some embodiments, the viewing window may be between about 0.5 mm and about 1.5 mm thick. For example, in some embodiments, the viewing window may be between about 0.8 and 1.0 mm thick. In some embodiments, a thickness of the body (e.g., the sides of the body) may be between about 1.0 mm and about 3.0 mm thick, such as about 2.0 mm thick. In some embodiments, the viewer may be between about 5 mm and about 10 mm tall (e.g., between the top of the viewing window and the bottom or second end of the viewer). For example, in some embodiments, the viewer may be between about 7.0 mm and about 8.0 mm tall.

As with the above and as shown in FIG. 3A, the device includes a securement feature 48 at the attachment end of the viewer to interface with the ascending aorta. In this embodiment, the viewer also includes at least one port 49 that connects the lumen of the valve viewer device to the atmosphere or to a fluid source. As also shown in this view, in some embodiments, the ports 49 may be positioned on a side of the viewer such that the ports do not obstruct viewing of the valve(s) via the viewing window. In some embodiments, as shown in this view, the ports may be located along different portions of a circumference of the first, end of the body. The ports also may be located at or near the first end of the body.

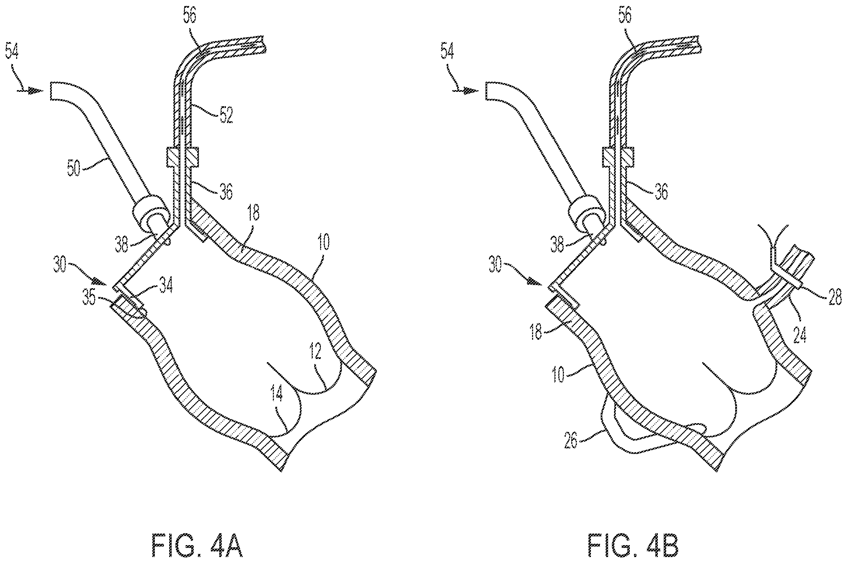

Referring to FIG. 4A, the viewer 30 is shown in a partial cross section with a body 34 of the viewer interfacing at the ascending aorta 18 which is contiguous with aortic root 10. As with the above, the securement feature 35 of the viewer also may interface with the ascending aorta 18 in such an arrangement. As shown in FIG. 4A, the viewer 30 has two ports attached to the body. A first port 38 is connected to a first portion of tubing 50 while second port 36 is connected to s second, separate portion of tubing 52. In such embodiments, tubing 50 may allow for fluid flow 54 into that tubing through the port and into the aortic valve viewer 30, which may then flow into the ascending aorta 18 and the aortic root 10 for pressurization. In such embodiments, the first part may be used to evacuate fluid from the aortic root, the ascending aorta, and the interior of the viewer.

In some embodiments, as shown in these views, the second port 36 may be positioned such that the port is at the highest point of the viewer 30. In such embodiments, the port may be positioned at the first end of the viewer. For example, the first port may be located on a side portion of the body, such as where the side of the body joins the first end of the body (e.g., at the viewing window). In other embodiments, the second port may be attached to the viewing end, such as to the viewing window. In such embodiments, the port may be attached away from a center of the viewing window, so as to not obstruct viewing of the valve(s).

In practice, when the viewer 30 is utilized, the viewer may be secured (e.g., reversibly) to the ascending aorta 18 after the ascending aorta has been opened and the aortic valve evaluated and possibly intervened upon. As will be appreciated, at this point during the intervention, the aortic root contains mostly air. To evaluate the aortic valve under pressure via the viewer, a fluid may be introduced into the aortic root 10 to distend the aortic root and cause a physiologically appropriate load on the aortic valve leaflets. The fluid may, thus, be used to distend the ascending aorta 18 and aortic root 10 and to impart load on the leaflets 12 and 14. In some embodiments, the fluid may include air, carbon dioxide, saline, plasmalyte, cardioplegia solution without blood, cardioplegia solution with a small amount of blood added in, and/or del Nido cardioplegia solution. Other suitable fluids may be used in other embodiments, as will be appreciated.

If embodiments in which the fluid used to fill the ascending aorta 18 or aortic root 10 is a liquid, the air within the ascending aorta and aortic root needs to be displaced to avoid an air liquid interface, which may distort the view of the aortic valve. In some embodiments, the air may be evacuated through the second port 36 with accompanying tubing 52. In such embodiments, flow of that fluid (see arrows 56 in FIGS. 4A and 4B), which may include a combination of air and the liquid fluid inserted into the ascending aortic root, could be evacuated to an adequate amount where all the air was evacuated from the ascending aorta and aortic root. Once the surgeon has adequately evacuated the air, the flow out the secondary port 36 and associating tubing 52 may be interrupted, and additional fluid flow through the first port 38 and associating tubing 50 could be used to increase the pressure within the aortic root and ascending aorta to a desirable level. In some embodiments, the level of pressure may be directly measured at or proximal to the second port 36. In some embodiments, additional ports within the viewer may be used directly, and in some embodiments continuously, to measure the pressure in the viewer during the time of evaluation of the aortic valve.

FIG. 4B illustrates the coronary arteries 24 and 26 that come off the aortic root 10 of the aorta to which the viewing device is attached. In some embodiments, flow 54 of fluid may be utilized to fill the aortic root and ascending aorta via port 38, with excess fluid and air being evacuated through port 36 and with that flow 56 persisting until the liquid fluid fills the ascending aorta or aortic root adequately. In such embodiments, the coronaries 24 and 26 coming off the aortic root may be generally open. Accordingly, when the aortic root 10 is pressurized there may be flow through those coronary arteries 24 and 26 even though the heart is arrested at this time during the intervention.

Flow through those arteries may be managed in a number of ways to adequately pressurize the aortic root 10 for evaluation of the aortic valve. In one embodiment, the coronary arteries may be temporary occluded with a tourniquet 28 or other means such as a temporary clip, a clamp, forceps, direct finger pressure, or any other surgically acceptable methods. As will be appreciated, temporary occlusions of one or more coronary arteries may allow for pressure build up to an acceptable degree within the aortic root 10 and avoid flow of fluid down the coronary arteries. This may be exceedingly important in situations where the fluid used to fill the aortic root 10 was a gas such as air, which a surgeon would not want to be infused in any degree down the coronary arteries. In another embodiment, the coronaries 24 and 26 may be left open while the aortic root 10 is pressurized, such as if the fluid used in the pressurized the aortic root is an acceptable fluid to transfuse to the coronary artery, such as cardioplegia solution. In one embodiment, del Nido cardioplegia solution without blood may be utilized as the fluid to pressurize the aortic root 10. In this case, there may be a constant low flow of fluid 54 through port 38 to allow for achievement of relatively steady-state pressure in the aortic root 10 accounting for some flow going out the coronary arteries 24 and 26. In another embodiment, one or more of the coronary arteries can be made partially or completely occluded by the surgeon's fingers, for example, to minimize the flow up the coronaries, necessitating a very low continuous fluid infusion through the coronaries to achieve adequate pressure in the aortic root 10.

In some embodiments, the viewing window may have different arrangements for viewing the valves (see, e.g., the viewing windows of the viewers 40 in FIGS. 5A-5E). In some embodiments, the viewing window may be arranged to allow for improved assessment of the valves, such as by magnifying the size of the valves, providing a wide-angle view, and/or reducing glare. For example, in pediatric patients, magnification of the view may be important, where ethe size of the valves being intervened on, and later assessed via the viewer, are small.

As shown in FIG. 5A, in some embodiments, the viewing window 32 may be substantially flat on its upper and lower surfaces, with the viewing window imparting no degree of magnification of the aortic valve when the user looks through the aortic valve viewer window 32. In such embodiments, the upper and lower surfaces of the viewing window may extend substantially parallel to one another. As shown in FIG. 5B, the viewer 40 also may have a viewing window 60 with a convex shape on the outer and inner surfaces of the window. In such embodiments, there may be a magnification effect to the viewer window 60 to provide a magnified view of the aortic valve by the user. As shown in this view, and as with FIG. 5A, the inner and outer surfaces of the window also may extend substantially parallel to one another, although in other embodiments, the curvature of the inner surface may differ from that of the curvature of the outer surface.

As shown in FIG. 5C, the viewer 40 also may have a viewing window 62 where the outer surface of the window is convex, and the inner surface is relatively flat. This arrangement also may provide some degree of magnification of the aortic valve for the user. As shown in FIG. 5D, the viewer 40 may include a window 64 where the inner and outer surfaces the aortic valve viewer window are concave. In this embodiment, the window 64 may provide a wide-angle view of the aortic valve for the surgeon. In such embodiments, the inner and outer surfaces of the window may extend substantially parallel to one another, although the curvature of the inner surface may differ from the curvature of the outer surface in some embodiments.

Referring to FIG. 5E, the viewer 40 may have a viewing window 66 where the outer surface of the aortic valve viewer window 66 may be concave and the inner surface may be flat. This arrangement may provide some degree of a wide-angle view of the aortic valve for the surgeon.

In some embodiments, the viewing window may be permanently attached to each viewer. In such embodiments, the user may select the desired viewer for the assessment prior to attaching the viewer to the aorta, after which time the aorta may be pressurized for viewing. In other embodiments, the viewing window may be removably attachable to the viewer. In such embodiments, the window may be changed during an assessment, such as if the surgeon is not able to fully visualize the valve(s) during pressurization. In such embodiments, the viewing window may be attached to a cap that is attachable to the body of the viewer (e.g., via snap fit, press fit, threading, or another suitable form of attachment).

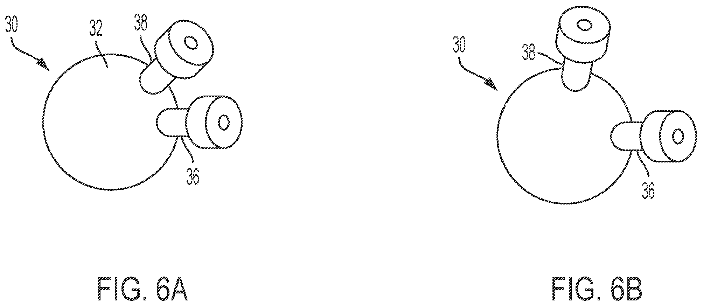

As previously described, the viewer may include one or more ports for fluid communication with the viewer. FIGS. 6A and 6B, top views of the viewer 30 with viewing window 32, illustrate different arrangement of these ports (see, e.g., port 36 and 38). In some embodiments, the fluid ports may exit the viewer at an angle that may vary from zero to ninety degrees relative to the plane of the viewing window 32. The fluid ports may be relatively spaced around the circumference of device, either closely spaced as shown in FIG. 6A or more widely spaced as is shown in FIG. 6B. For exmaple, an angle between the first and second ports may be between about 0 and 60 degrees, such as about 45 degrees in some embodiments. In some embodiments, the fluid ports 36 and 38 may serve as a handle for manipulating the position of the viewer in the patient.

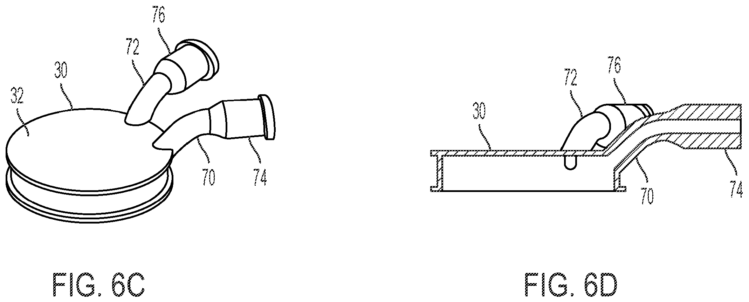

As shown in FIG. 6C, the fluid ports may have extensions 70 and 74 arranged to connect the body to lower connections 74 and 76. In some embodiments, the extensions 70 and 72 may be configured such that they are attached (e.g., come) straight off the aortic valve viewer 30. The extensions also may be angled relative to the plane of the viewing angle and come off curved in one or more planes. In some embodiments, the connectors 74, 76 include luer connectors and allow for quick attachment of tubing or syringes to the aortic valve viewer 30.

As shown in FIG. 6D, the fluid connectors with the extension 70 and luer connecter 74 have a fluid pathway that communicates with the interior of the aortic valve. In some embodiments, this arrangement may allow for perfusion of clear fluid into the viewer via one port and exit of excess fluid and air out of the opposite port in one utilization of the device. The fluid ports also may be used to introduce air into the aortic root for visualization. In some embodiments, the fluid ports may be used to introduce cardioplegia into the aortic root for introducing of cardioplegia down the coronary arteries.

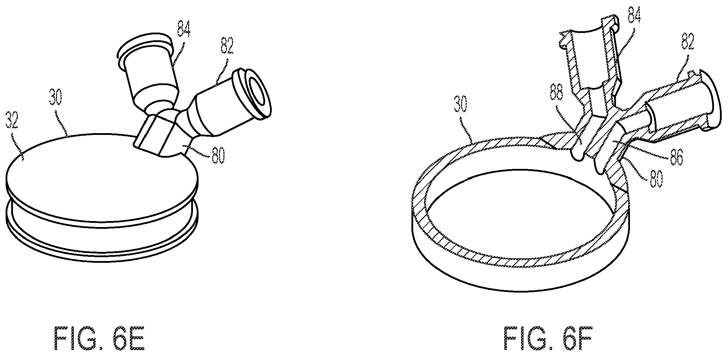

In another embodiment shown in FIG 6E, the viewer 30 may have a coupler 80 where the fluid ports 82 and 84 are joined together before entering the aortic valve viewer. A cross sectional view of the viewer is shown in FIG. 6F, which shows the first and second pathways of the joined fluid ports in the coupler. For example, fluid pathway 86 that goes through the luer connector 82 and fluid pathway 88 that goes through port 84 to allow fluid to communicate with the interior of the viewer 30 are both in the coupler 80.

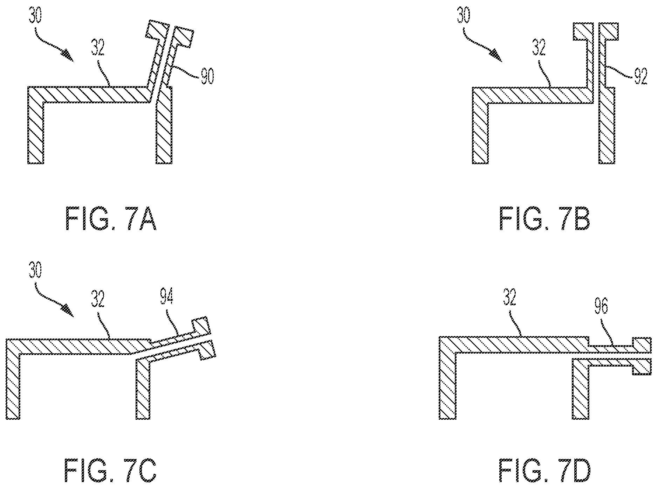

Another embodiment of the viewer is shown in FIG. 7A, where the aortic valve viewer 30 has a viewing window 32 and a fluid port 90 that intersects with the aortic valve viewer. As shown in this view, the fluid port 90 may extend at an acute angle relative to the viewing window 32. In another embodiment, as shown in FIG. 7B, one or more fluid ports 92 may extend at an angle substantially perpendicular to the viewing window 32. In the embodiment shown in FIG. 7C, the viewer 30 may have one or more fluid ports 94 that extend at an angle relative to the viewing window. In some embodiments, the port may be angled between about 15 degrees and about 60 degrees, such as about 30 degrees. The ports 96 also may extend substantially parallel to the viewing window 32, as shown in FIG. 7D.

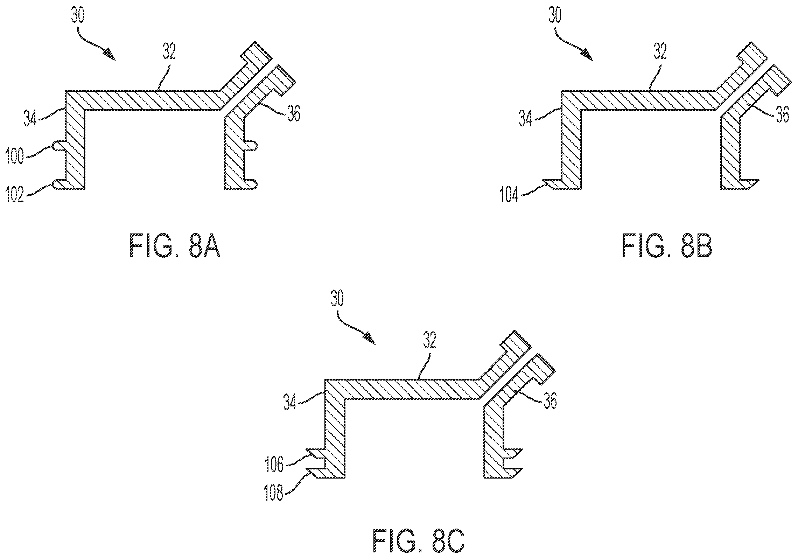

Another embodiment of the valve viewer 30 is shown in FIGS. 8A-8C, with the viewer having a viewing window 32, a cylindrical body 34, and one or more fluid ports 36 communicating with the interior of the device. As with the above, the viewer may be reversibly secured to the aorta or the pulmonary artery via a portion of the body. As shown in these views, the device may include one or more attachment features to facilitate attachment of the device to the aorta. As shown in FIG. 8A, the attachment features may be located on the outside of the body and may be curved in cross section and adequately spaced about the circumference of the body to provide reversible securement to the aorta via tourniquet that is tightened between the features 100 and 102. In another embodiment, as shown in FIG. 8B, the cylindrical body of device 34 may have a single attachment feature located at or near the portions of the body that interfaces with the aorta (e.g., at or near the second attachment end of the body). This external feature may have an angled portion (e.g., like a barb) to facilitate insertion into the aorta. In another embodiment, as shown in FIG. 8C, one or more closely spaced attachment features 106, 108 at or near the portions of the body 34 interfacing with the aorta (e.g., at or near the second attachment end of the body). In some embodiments, these attachment features 106, 108 also may have an angled portion to enhanced securement of the device to the aorta.

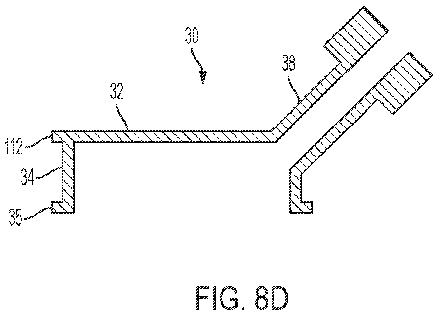

As shown in FIG. 8D, the aortic valve viewer 30 may include multiple external attachment features on the body 34, with features located both at or near the first viewing end of the viewer and at or near the second attachment end of the viewer. In some embodiments, attachment features 112 may be located at or near the level of the viewing window. In some embodiments, securement may be achieved by an external compression between the attachment features. The attachment feature 112 may be similar or different in size to the feature 35 in some embodiments. In some embodiments, feature 112 may have a larger diameter than that of other features to provide some limitation of the depth of the insertion of the aortic valve viewer into the aorta to prevent the aortic valve viewer from slipping into the aorta (e.g., as a stopper). Features 35 and 112 may have a rounded cross-sectional area or may have an angle to the cross-sectional area to facilitate insertion and or limitation of movement into the aorta.

In some embodiments, the attachment features include barbs, pins, or other suitable attachment features for inserting into the aorta, although other features may be used.

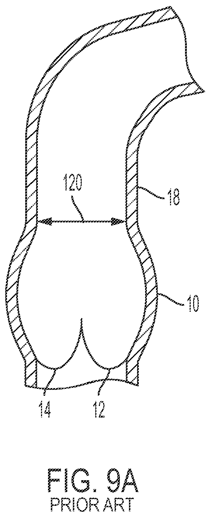

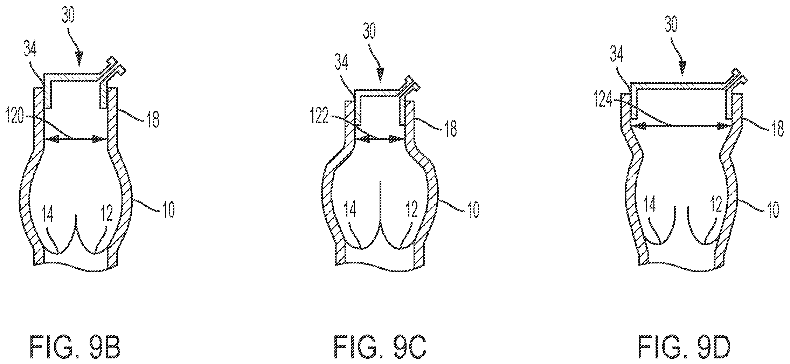

Utilizing the aortic valve viewer in the ascending aorta to visualize the aortic valve may involve securement of the viewer to the aorta. FIG 9A shows an aortic root 10 with ascending aorta 18 and sinotubular junction 1. Typically, the aorta is opened distal to the sinotubular junction 120 in an area in the ascending aorta 18 to give the surgeon access to visualize the aortic valves leaflets 12 and 14. FIG. 9b shows the aortic root 10 with ascending aorta 18 and sinotubular junction 120, with a viewer 30 positioned within the ascending aorta 18 and with a body 34 of the viewer (e.g., a cylindrical body) positioned adjacent to the ascending aorta and at least partially within the ascending aorta 18.

As shown in FIG. 9B, the valve viewer body 34 (e.g., an outer diameter of the second end) may be sized to be the same or similar to the internal diameter of that portion of the ascending aorta 18 and sinotubular junction 120. As represented in this figure, sizing of the viewer to match the size of the ascending aorta and sinotubular junction results in minimal to no stretching or compressing of the ascending aorta or sinotubular junction, which may avoid distortion of the aortic valve while the aortic root is pressurized and the aortic valve is visualized.

As shown in FIG. 9C, if the viewer body 34 is smaller than the ascending aorta 18 when the ascending aorta 18 is secured to the viewer body 34, a reduction of the size of the sinotubular junction (see arrow 122) may occur, which may distort the aortic valve leaflets 12 and 14. This may give inaccurate information about the performance of the aortic valve once the patient has been weaned off bypass and the ascending aorta and the sinotubular junction are back to their physiologic sizes while pressurized. As shown in 9D, if the viewer 30 has a body portion 34 with a diameter that is larger than the native internal ascending aorta diameter 18, a significant stretch of the sinotubular junction area (see arrow 124) may occur, as can subsequent distortion of the aortic valve of the aortic root 10 and separation of the leaflets of the aortic valve 12 and 14. Thus, using a viewer in the ascending aorta that is too large also may result in incorrect in evaluation of the aortic valve. Accordingly, desired sizing of the viewer according to embodiment disclosed herein, include a viewer that can pressurize the aortic root without substantial distortion of the aortic root or aorta compared to how the structures would be during normal physiologic function. Such a viewer may provide an accurate assessment of the aortic valve while the heart is still stopped.

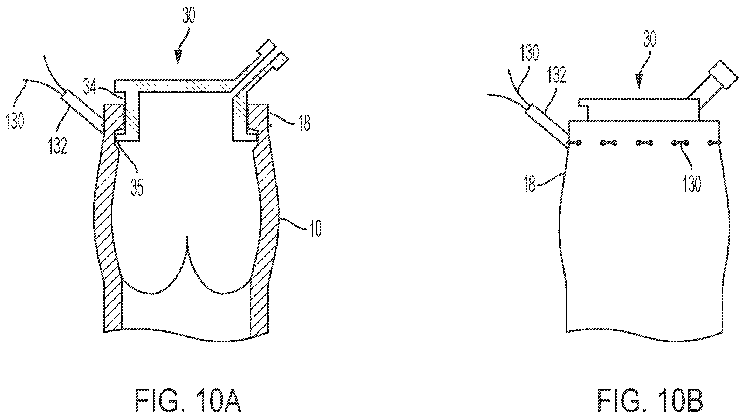

FIG. 10A shows an embodiment in which the aortic valve viewer 30 has a cylindrical body 34 positioned within the ascending aorta 18 and above the aortic root 10. As shown in this view, an external attachment feature 35 aids in the securement of the valve viewer to the ascending aorta 18. In some embodiments, the attachment feature may extend outwardly from the side of the body. In some embodiments, the attachment feature extends substantially perpendicular to the body. In some embodiments, a suture 130 may be placed around the ascending aorta at the level where the aortic valve viewer is inserted into the ascending aorta. This suture may pass through a tourniquet 132 which allows the aorta to be tightened around the aortic valve viewer and provide a fluid seal. As shown in FIG. 10B, the suture 130 may be reversibly returned to the aorta through a partial thickness bites on the aorta or simply be positioned on the outside of the aorta and passed through the tourniquet 132. This can help seal the aortic valve viewer 30 to the ascending aorta 18.

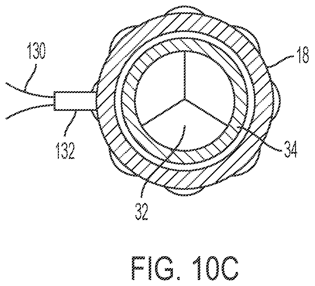

Other securement methods may be used rather than a suture, including a clamp, zip tie, vessel loop, or holding the aortic valve viewer securely adjacent to the aorta. FIG. 10C shows a top view of the viewer and viewer body looking down through the viewer window to the aortic valve. As shown in this view, the aortic purse string suture 130 is shown passing through the tourniquet 132, securing the aorta 18 to the cylindrical portion of the aortic valve viewer 34. The aortic valve is visible in a pressurized state below the aortic valve viewer.

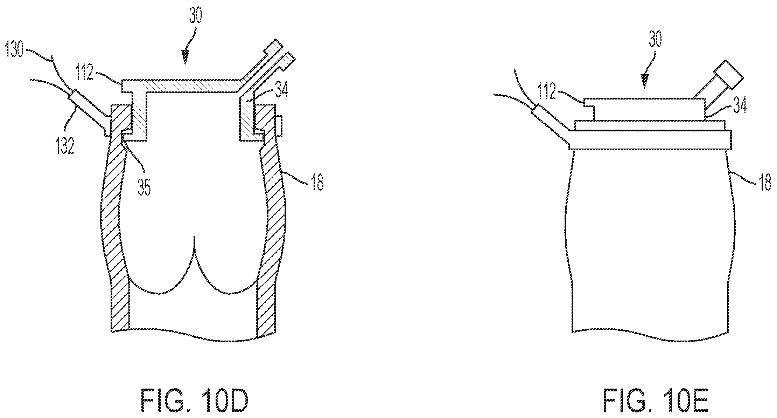

FIG. 10D shows another embodiment where the viewer 30 is reversibly secured to the ascending aorta 18. As shown in this view, the suture 130 may be secured to the ascending aorta via tourniquet 132, which may compress the aorta in between the attachment features 35 and 112. In some embodiments, the feature 112 may act as a stopper to prevent the viewer from slipping into the aorta, while features 35 may act as a stopper to prevent the viewer from slipping out of the aorta while reversibly secured. FIG. 10E is an external view of the ascending aorta 18 of FIG. 10D, with the viewer 30 reversibly secured in place. As shown in this view, the stopper extends above the top of the aorta and acts to prevent the viewer from slipping into the aortic valve, once secured.

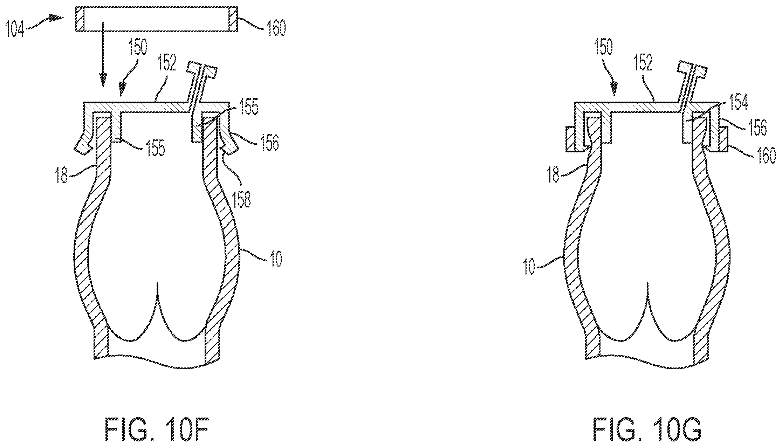

FIGS. 10F and 10G shows another embodiment in which the viewer 150, having body 154 and viewer window 152, is secured to the ascending aorta 18. As shown in this view, a portion of the aorta is positioned in between an inner body portion 155 and an outer body portion 156. In some embodiments, the inner body portion may include a cylindrical member that is inserted inside the aorta. In such embodiments, the outer body portion may be biased in an open position (see FIG. 10F), such that a portion of the aorta may be slid in between the inner and outer body portions when the viewer is placed on the aorta. In some embodiment, the outer body portion includes an attachment feature 158, such as a barb, that may extend inwardly toward the aorta and towards the inner body portion and that may aid in gripping the ascending aorta tissue when the viewer is secured to the aorta.

As shown in FIG. 10F, a securement ring may be placed over the outer body portion 156 (see arrow) to move the outer body portion towards the inner body portion 155. Once the ring is in place, the aorta may be held between the inner and outer body portions to secure the viewer to the aorta. FIG. 10G shows the aortic valve viewer 150 with the securement ring 160 in place, the outer body portion in a closed or secured position, and the viewer secured to the aorta.

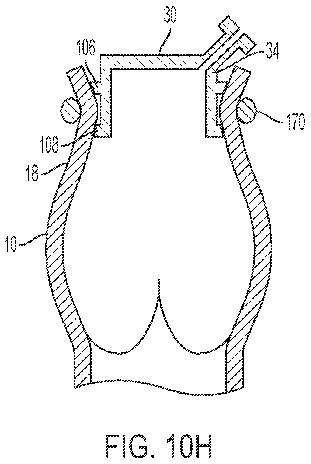

FIG. 10H shows still another embodiment of the viewer 30 secured to the aorta. As shown in this view, the body includes attachment features 106 and 108. In this embodiment, a band 170 may be positioned around the aorta to hold the aorta against the body, in between the first and second attachment features. In some embodiments, the attachment features may include barbs that are insertable into a portion of the wall of the aorta to assist in gripping the aorta when the band is in place. In some embodiments, the band 170 may be formed of an elastic material (e.g., an elastic band) or be another type of band that is closable around the aorta to secure the viewer to the aorta.

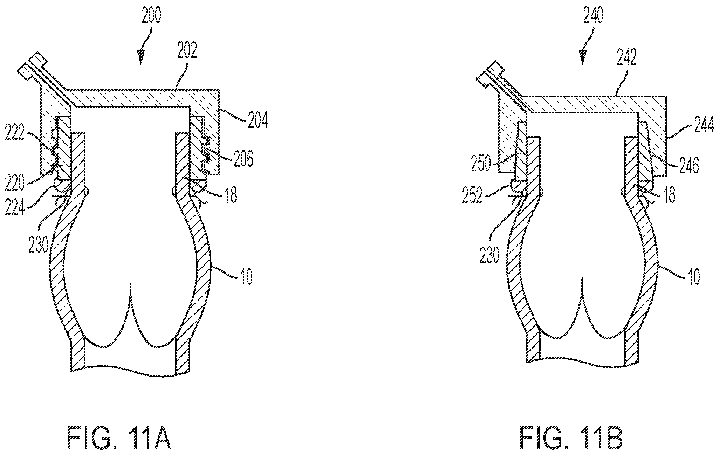

FIG. 11A shows another embodiment of the aortic valve viewer 200 being secured to the aorta. As shown in this view, the inner surface of the body 204 includes threads 206 arranged to engage with an inner securement ring 200 with corresponding threads 222. In some embodiments, the securement ring 220 includes one or more attachment features 224 to attach the ring to the aorta. For example, the securement ring 220 may be attachable to the ascending aorta 18 via suture 230. As shown in FIG. 11A, the body 204 of the device may be secured to the aorta by threading the body the internal securement ring 220 which is first placed over the ascending aorta 18.

In some embodiments, the viewer 200 may be formed in a single size, with the securement ring being formed with differently sized inner diameters to match the size of the patient's aorta. In such embodiments, the surgeon may select the appropriately sizes inner securement ring 220 and attach the securement ring to the ascending aorta via suture 230. The necessary watertight seal may thereafter be provided by threading the body 204 of the viewer 200 onto the inner securement ring 220.

FIG. 11B illustrates another example in which the body of the viewer is attachable to an inner securement ring 250 that is, in turn, attached to the aorta. As shown in FIG. 11B, instead of a threaded engagement between the body and inner securement ring, the body 244 of the viewer 240 may be attached inner securement ring via a press fit engagement. For example, as shown the body 244 may include an angled internal surface that reversibly engages with the corresponding angled internal surface 236 of the inner securement ring 250. As with the above, the inner securement ring may be attachable (e.g., reversibly attachable) to the ascending aorta by suture 230. Also similar to the above, the press fit engagement may form a seal by which the ascending aorta can be fluid filled and pressurized to evaluate the aortic valve.

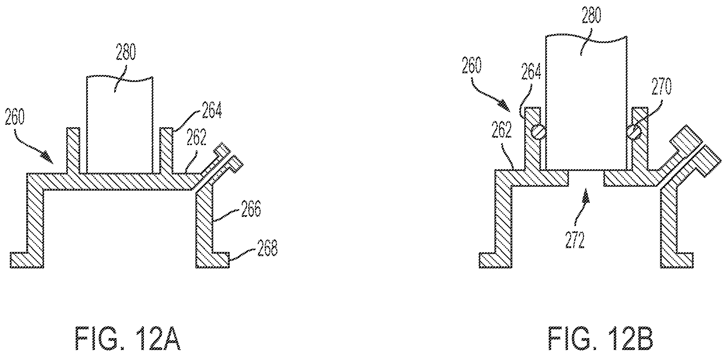

In some embodiments, the viewer may be used with a viewing scope or camera to assist with or supplement the visualization of the viewer. For example, the viewing scope or camera 280 may include a laparoscopic-type scope that has a camera at the back or it may be an individual camera that is applied directly onto the aortic valve viewer. As shown in FIG. 12A, in some embodiments, the viewer 260, may include a viewing window 262 and extensions 242 above the viewing window. In such embodiments, the viewer also may include a tubular body and one or more securement features (e.g., securement feature 268).

As shown in FIG. 12A, the viewing scope or camera 280 may be advanced up to the level of the aortic valve viewer window 262, with the extensions positioned around at least a portion of the scope and/or camera (e.g., a distal end of the scope and/or camera). In such embodiments, the scope or camera may allow for visualization for the aortic valve through a scope or camera rather than visualizing it with direct visualization. In some embodiments, this approach may be useful in recording the function of the aortic valve for later inspection or analysis.

In some embodiments, the extension may be arranged to provide some securement for the scope and/or camera. For example, in some embodiments, the distance between the extensions (e.g., length or diameter) may be sized fit the camera and/or scope. In such embodiments, the scope and/or camera may have a close enough fit with the extensions such that the extensions hold the scope or camera to the viewer. In other embodiments, the extensions may include one or more securement features, such as features 270, to assist in securing the camera and/or scope in the extensions. For example, the features may create a frictional or interference fit between the end of the camera and/or scope and the extensions. The features may have other suitable arrangements in other embodiments. As will be appreciated, although the viewer is shown with extension portions and additional securement features, in some embodiments, the viewing scope and/or camera may be simply placed against the viewing window of the viewer.

In some embodiments, the viewer may be used with a camera or scope viewing element looking directly through the viewing window and viewing lens of the camera or scope. In another embodiment shown in FIG. 12B, the viewing window 262 may have an opening 272 which allows for direct access to the internal portion of the aortic valve viewer device. In some embodiments, without this viewing window, there may need to be a hook-up interface between the camera and scope to minimize any distortion of the image. In some embodiments, the viewing window 272 may provide direct visualization and possibly a clearer picture in visualizing the aortic valve. In this embodiment the extension portions 264 may include a securement member 270 that may interface with the scope or camera element to 280 to provide a fluid seal so that the space inside the aortic valve can be pressurized for distinction of the aortic root and visualization of the aortic valve under pressure.

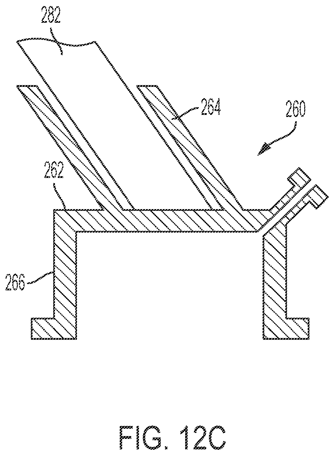

FIG. 12C illustrates another example in which a scope or camera 282 may be secured to the viewer. As shown in this view, the securement features may include extensions 264 that extend from the aortic valve window 260 at an angle. As also shown in this view, there may be two extensions at an angle to allow interface with the scope that has an angled viewing lens on it. In some embodiments, such a viewer with an angled scope interface may be more ergonomically appropriate given the constraints of using this within the open chest as part of a cardiac surgical procedure.

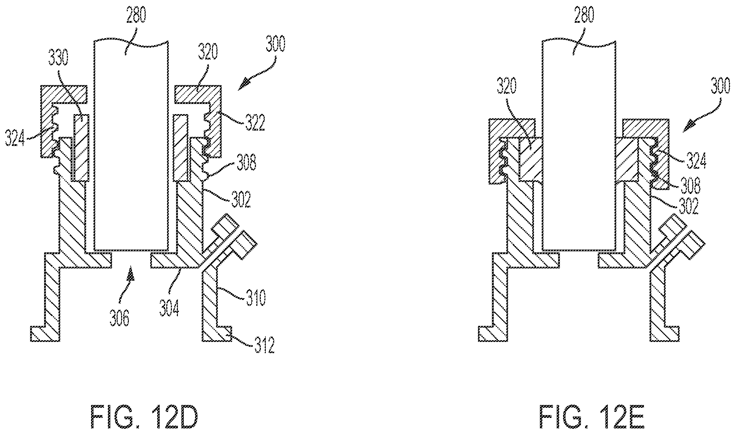

In another embodiment, the viewer may include a valve that engages with a scope or camera member inserted into the viewer (see FIG. 12D). In this embodiment, the viewer 300 may include a cylindrical extension 302 that extends from the aortic valve viewing window 304 which may have an opening 306 in the window. A thread 308 on the cylindrical portion 302 may provide a threaded interface with a second, outer portion of the viewer 320, which includes a cylindrical portion 322 and corresponding threads 324. As these two portions, outer portion 320 and extending member 302, are threaded together they can compress a flexible member 330 which may then reversibly engage with the scope or camera element 280 to provide a seal (see FIG. 12E). In this embodiment the flexible member 320 is compressed by the upper portion 320 and the extending member 302 to form a seal against the camera member 280. This may be similar to the Tuohy-borst valve which is commonly used to provide a reversible seal.

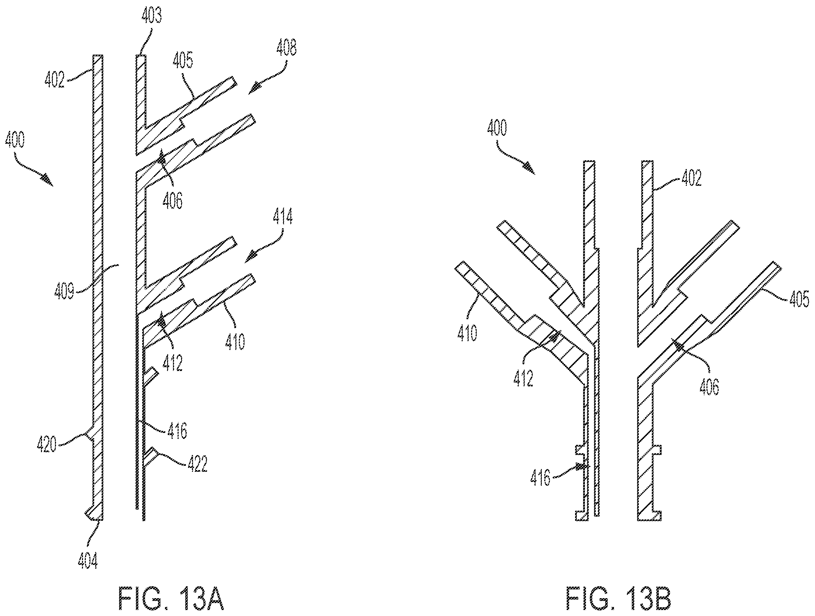

In some embodiment of the viewer, there may exist a need to at times visualize the aortic valve when the aorta has not been completely divided or when the ascending aorta and aortic valve are substantially smaller in diameter, as in the case of infant and pediatric aortic valve interventions. For these applications, an embodiment of the valve viewer that utilizes a camera may facilitate visualization of the aortic valve. FIG. 13A-13E show embodiments in which the viewer 400 that has a cylindrical body 402 with a first end 403 for visualization and a second 404 for attachment. In some embodiments, as shown in FIG. 13A, the viewer may include a connector 406 and associated connector 408 that communicates with the lumen of the viewer 409. In some embodiments, there is a second fluid connector 410 with lumen 412 and more lower connector 414 which communicates with the secondary lumen 416 and which also provides fluid communication into the aorta. The device may include one or more external securement features 420 and 422 which may facilitate reversible securement of the viewer to the aorta.

In another embodiment shown in FIG. 13B, the viewer 400 may have fluid ports, fluid port 405 and lumen 406, which may be arranged opposite to the second fluid port 410 with fluid lumen 412 communicating with secondary lumen 416. These fluid ports 405 and 410 as well as the first end of the 402 may have slip-luer type connections that facilitate connecting fluid connections to the viewer. Fluid introduced via the fluid ports may pressurize the aortic root and another port may deair the aortic root to facilitate visualization of the aortic valve under physiologic pressure.

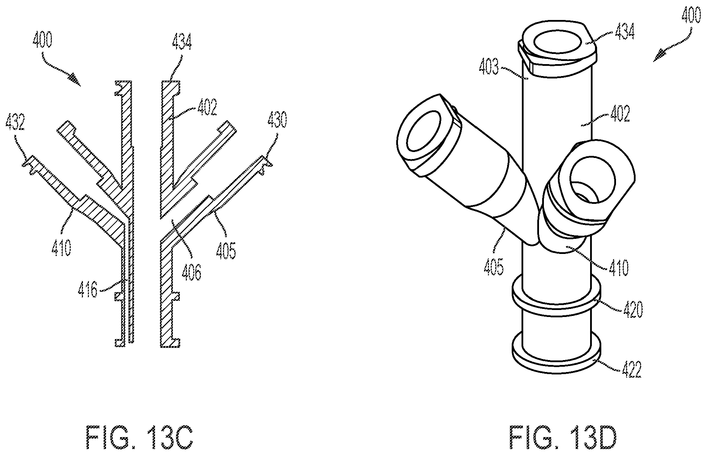

In another embodiment, as shown in FIG. 13 C, the viewer 400 may have a cylindrical body 402 with fluid ports of 405 and 410, which have lower type connectors 430 and 432. In some embodiments, the first end of the valve may have a lower connector 434, although the first end may not have such a lower connector in other embodiments. In some embodiments, the lower connector 434 may be able to accept a Tuohy-borst style valve that could provide seal to a camera that could extend through the lumen of the viewer. FIG. 13D shows is an isometric view of the embodiment of the aortic valve viewer 400 with a cylindrical body 402 and fluid ports 405 and 410, as well as the external securement features 420 and 422 which can be used to secure to the wall of the aorta.

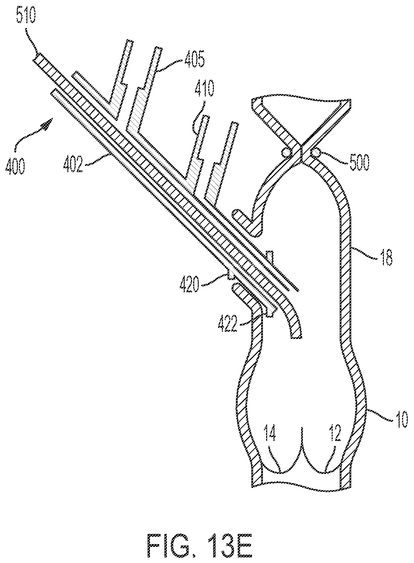

FIG. 13E provides a cross-sectional view of the 400 that is engaged with ascending aorta 18. In this embodiment the tubular body of the viewer 402 has a viewing scope 510 advanced through the body of viewer and into the ascending aorta 18, which provides visualization of the aortic root 10 and the aortic valve leaflets 12 and 14. The ascending aorta is occluded distally with cross-clamp 500. This may allow for introduction of fluid into one of the ports 405 or 410 and aspiration of fluid out of the other port to provide de-airing and pressurization of the ascending aorta and aortic root for visualization of the aortic valve under pressurized conditions. The camera 510 in this embodiment may be a flexible scope that is attached to a camera at the most proximal end. This may allow the surgeon to visualize the aortic valve via the camera.

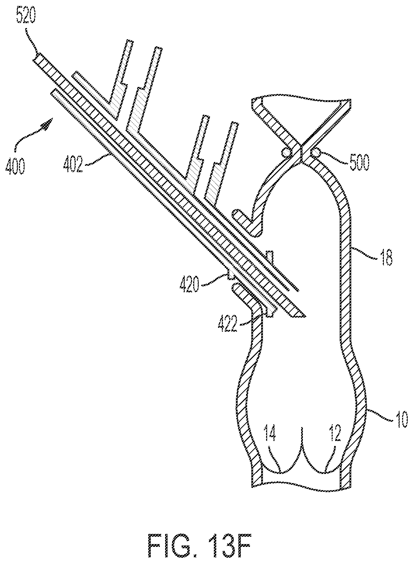

In another embodiment, shown in FIG. 13F, the aortic valve viewer 400 has a tubular body 402 that allows advancement of a scope 520 through the aortic valve viewer and into the ascending aorta 18. In this embodiment, the scope is a straight scope with an angle at the end, which allows an angle view of the aortic valve so the aortic valve leaflets 12 and 14 can be inspected. Laparoscopes at 30° and 45° angles that are commonly used in laparoscopic and thorascopic surgery and this type of scope could be utilized with the aortic valve viewer in this type of application for visualization of the aortic valve.

In some embodiments, the entire viewer may be formed of a material that is transparent. In some embodiments, the viewer may be formed of a rigid material. For example, the viewer may be formed of a plastic material. In some embodiments, the viewer is formed of an acrylic or polycarbonate material. In such examples, the viewer may be arranged to be sterilized at high temperatures, allowing the viewer to be reusable. The viewer also may be formed to be disposable in some embodiments. In some embodiments, the viewing window is arranged to be formed of a materially that is optically clear.

In some embodiments, at least a portion of the viewer may be formed of a flexible material. For example, in some embodiments, the body may be collapsible. In such embodiments, the viewing window may include a rigid disc that is attachable to the collapsible tubular body. As will be appreciated, the body may be expanded for attachment to the aorta.

In some embodiments, the viewer is integrally formed. For example, the body, viewing, window, and ports are integrally formed. In other embodiments, the viewer may include one or more parts that are attachable to one another. In some embodiments, the multiple components are fixedly attached to each other, while, in other embodiments, one or more pieces may be removably attachable to one another.

While the present teachings have been described in conjunction with various embodiments and examples, it is not intended that the present teachings be limited to such embodiments or examples. On the contrary, the present teachings encompass various alternatives, modifications, and equivalents, as will be appreciated by those of skill in the art. Accordingly, the foregoing description and drawings are by way of example only.

Various aspects of the present invention may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

It should be appreciated that the present invention can be implemented and utilized in numerous ways, including without limitation as an apparatus, system, device, a kit (e.g., a kit comprising one of the platforms described herein in this first-of-use), and for applications now known and later developed. These and other unique features of the system disclosed herein will become more readily apparent from the following description and the accompanying drawings.

It is to be understood that the subject technology is not intended to be limited to the particular constructs and methods described in the described embodiments, as one skilled in the art can extend the concepts involved using variations which are obvious after reading the present disclosure. Although any methods and materials, similar or equivalent to those described herein, may be useful in a practice of the subject technology, certain compositions, films, methods, and materials are described below. All relative descriptions herein, such as "top," "bottom," "left," "right," "up," and "down" are with reference to the figures, and not meant to be in a limiting sense.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Also, the phraseology and terminology used herein is for the purpose of description and should not be regarded as limiting. The use of "including," "comprising," "having," "containing," or "involving," and variations thereof herein, is meant to encompass the items listed thereafter as well as additional items.

## Claims

1. A viewer (40) comprising;
a body (42, 44, 46) having a first end and a second end opposite the first end, the first end being closed and having a transparent viewing window (32, 41, 60, 62, 64, 66), the second end arranged to be attached to an aorta or a pulmonary artery, wherein an outer diameter of the first end of the body is larger than an outer diameter of the second end of the body, wherein the body includes one or more securement features (48) including a rim extending outwardly from the second end, and wherein the rim is arranged to permit attachment of the second end to the aorta or the pulmonary artery; and
one or more ports (49) attached to the body and arranged to provide fluid access to an internal portion of the viewer.

2. The viewer according to claim 1, wherein the body includes a tubular body.

3. The viewer according to any one of the preceding claims, wherein a first outer diameter of a first portion (42) of the body is greater than a second outer diameter of a second portion (46) of the body, the first portion of the body being closer to the first end than the second portion of the body is to the first end.

4. The viewer according to claim 3, wherein the body includes a tapered portion (44) positioned in between the first portion (42) of the body and the second portion (46) of the body.

5. The viewer according to claim 4, wherein a first outer diameter of a first end of the tapered portion (44) is the same as the first outer diameter of the first portion (42) of the body and a second outer diameter of a second end of the tapered portion (44) is the same as the second outer diameter of the second portion (46) of the body.

6. The viewer according to claim 1, wherein the body includes a tapered portion (44), wherein an outer diameter of the tapered portion decreases in a direction from the first end of the body towards the second end of the body.

7. The viewer according to claim 6, wherein the outer diameter of the tapered portion (44) decreases from the first end to a portion in between the first and second ends.

8. The viewer according to any one of the preceding claims, wherein the one or more ports (49) extend outwardly from a side of the body.

9. The viewer according to any one of the preceding claims, wherein the one or more ports are located at or near the first end of the body.

10. The viewer according to any one of the preceding claims, wherein the one or more ports are fixedly attached at the first end of the body.

11. The viewer according to any one of the preceding claims, wherein the body is attachable to the aorta or pulmonary artery via the one or more securement features.

12. The viewer according to any one of the preceding claims, further comprising one or more extensions (264; 302) extending outwardly from the viewing window for engaging with a camera or scope (280; 282).

13. The viewer according to any one of the preceding claims, wherein a diameter of the viewing window is greater than the outer diameter of the second end.

14. The viewer according to any one of the preceding claims, wherein an inner diameter of the body is uniform between the first and second ends.

15. The viewer according to any one of the preceding claims, wherein an inner diameter of the first end of the body is greater than an inner diameter of the second end of the body.

## Patentansprüche

1. Sichtgerät (40), umfassend:
einen Körper (42, 44, 46), der ein erstes Ende und ein zweites Ende gegenüber dem ersten Ende aufweist, wobei das erste Ende geschlossen ist und ein transparentes Sichtfenster (32, 41, 60, 62, 64, 66) aufweist, wobei das zweite Ende so angeordnet ist, um an einer Aorta oder einer Pulmonalarterie befestigt zu werden, wobei ein Außendurchmesser des ersten Endes des Körpers größer ist als ein Außendurchmesser des zweiten Endes des Körpers, wobei der Körper eine oder mehrere Sicherungsmerkmale (48) einschließt, einschließlich eines Randes, der sich von dem zweiten Ende nach außen erstreckt, und wobei der Rand so angeordnet ist, dass er eine Befestigung des zweiten Endes an der Aorta oder der Pulmonalarterie ermöglicht; und
einen oder mehrere an dem Körper befestigte Anschlüsse (49), die so angeordnet sind, dass sie einen Flüssigkeitszugang zu einem internen Abschnitt des Sichtgeräts bereitstellen.

2. Sichtgerät nach Anspruch 1, wobei der Körper einen rohrförmigen Körper einschließt.

3. Sichtgerät nach einem der vorstehenden Ansprüche, wobei ein erster Außendurchmesser eines ersten Abschnitts (42) des Körpers größer ist als ein zweiter Außendurchmesser eines zweiten Abschnitts (46) des Körpers, wobei der erste Abschnitt des Körpers näher an dem ersten Ende als der zweite Abschnitt des Körpers an dem ersten Ende ist.

4. Sichtgerät nach Anspruch 3, wobei der Körper einen konischen Abschnitt (44) einschließt, der zwischen dem ersten Abschnitt (42) des Körpers und dem zweiten Abschnitt (46) des Körpers positioniert ist.

5. Sichtgerät nach Anspruch 4, wobei ein erster Außendurchmesser eines ersten Endes des konischen Abschnitts (44) derselbe ist wie der erste Außendurchmesser des ersten Abschnitts (42) des Körpers und ein zweiter Außendurchmesser eines zweiten Endes des konischen Abschnitts (44) derselbe ist wie der zweite Außendurchmesser des zweiten Abschnitts (46) des Körpers.

6. Sichtgerät nach Anspruch 1, wobei der Körper einen konischen Abschnitt (44) einschließt, wobei ein Außendurchmesser des konischen Abschnitts in einer Richtung von dem ersten Ende des Körpers in Richtung des zweiten Endes des Körpers abnimmt.

7. Sichtgerät nach Anspruch 6, wobei der Außendurchmesser des konischen Abschnitts (44) von dem ersten Ende auf einen Abschnitt zwischen dem ersten und zweiten Ende abnimmt.

8. Sichtgerät nach einem der vorstehenden Ansprüche, wobei sich die einen oder mehreren Anschlüsse (49) von einer Seite des Körpers nach außen erstrecken.

9. Sichtgerät nach einem der vorstehenden Ansprüche, wobei sich die einen oder mehreren Anschlüsse an dem oder in der Nähe des ersten Endes des Körpers befinden.

10. Sichtgerät nach einem der vorstehenden Ansprüche, wobei die einen oder mehreren Anschlüsse fest an dem ersten Ende des Körpers befestigt sind.

11. Sichtgerät nach einem der vorstehenden Ansprüche, wobei der Körper über die einen oder mehreren Sicherungsmerkmale an der Aorta oder der Pulmonalarterie befestigt werden kann.

12. Sichtgerät nach einem der vorstehenden Ansprüche, weiter umfassend eine oder mehrere Verlängerungen (264; 302), die sich nach außen von dem Sichtfenster erstrecken, um mit einer Kamera oder einem Endoskop (280; 282) in Eingriff zu kommen.

13. Sichtgerät nach einem der vorstehenden Ansprüche, wobei ein Durchmesser des Sichtfensters größer ist als der Außendurchmesser des zweiten Endes.

14. Sichtgerät nach einem der vorstehenden Ansprüche, wobei ein Innendurchmesser des Körpers zwischen den ersten und zweiten Enden gleich ist.

15. Sichtgerät nach einem der vorstehenden Ansprüche, wobei ein Innendurchmesser des ersten Endes des Körpers größer ist als ein Innendurchmesser des zweiten Endes des Körpers.

## Revendications

1. Dispositif de visualisation (40) comprenant :
un corps (42, 44, 46)
présentant une première extrémité et une deuxième extrémité opposée à la première extrémité, la première extrémité étant fermée et présentant une fenêtre de visualisation transparente (32, 41, 60, 62, 64, 66),
la deuxième extrémité étant agencée pour être fixée à une aorte ou une artère pulmonaire,
dans lequel un diamètre externe de la première extrémité du corps est plus grand qu'un diamètre externe de la deuxième extrémité du corps, dans lequel le corps inclut une ou plusieurs caractéristiques de fixation (48) incluant un rebord s'étendant vers l'extérieur à partir de la deuxième extrémité, et dans lequel le rebord est agencé pour permettre la fixation de la deuxième extrémité à l'aorte ou à l'artère pulmonaire ; et
un ou plusieurs orifices (49) fixés au corps
et agencés pour fournir un accès fluidique à une partie interne du dispositif de visualisation.

2. Dispositif de visualisation selon la revendication 1, dans lequel le corps inclut un corps tubulaire.

3. Dispositif de visualisation selon l'une quelconque des revendications précédentes, dans lequel un premier diamètre externe d'une première partie (42) du corps est supérieur à un deuxième diamètre externe d'une deuxième partie (46) du corps, la première partie du corps étant plus proche de la première extrémité que la deuxième partie du corps ne l'est de la première extrémité.

4. Dispositif de visualisation selon la revendication 3, dans lequel le corps inclut une partie conique (44) positionnée entre la première partie (42) du corps et la deuxième partie (46) du corps.

5. Dispositif de visualisation selon la revendication 4, dans lequel un premier diamètre externe d'une première extrémité de la partie conique (44) est identique au premier diamètre externe de la première partie (42) du corps et un deuxième diamètre externe d'une deuxième extrémité de la partie conique (44) est identique au deuxième diamètre externe de la deuxième partie (46) du corps.

6. Dispositif de visualisation selon la revendication 1, dans lequel le corps inclut une partie conique (44), dans lequel un diamètre externe de la partie conique diminue dans une direction depuis la première extrémité du corps vers la deuxième extrémité du corps.

7. Dispositif de visualisation selon la revendication 6, dans lequel le diamètre externe de la partie conique (44) diminue de la première extrémité à une partie entre les première et deuxième extrémités.

8. Dispositif de visualisation selon l'une quelconque des revendications précédentes, dans lequel les un ou plusieurs orifices (49) s'étendent vers l'extérieur à partir d'un côté du corps.

9. Dispositif de visualisation selon l'une quelconque des revendications précédentes, dans lequel les un ou plusieurs orifices se situent au niveau ou à proximité de la première extrémité du corps.

10. Dispositif de visualisation selon l'une quelconque des revendications précédentes, dans lequel les un ou plusieurs orifices sont fixés à demeure à la première extrémité du corps.

11. Dispositif de visualisation selon l'une quelconque des revendications précédentes, dans lequel le corps peut être fixé à l'aorte ou à l'artère pulmonaire via les une ou plusieurs caractéristiques de fixation.

12. Dispositif de visualisation selon l'une quelconque des revendications précédentes, comprenant en outre une ou plusieurs extensions (264 ; 302) s'étendant vers l'extérieur à partir de la fenêtre de visualisation pour venir en prise avec une caméra ou un endoscope (280 ; 282).

13. Dispositif de visualisation selon l'une quelconque des revendications précédentes, dans lequel un diamètre de la fenêtre de visualisation est supérieur au diamètre externe de la deuxième extrémité.

14. Dispositif de visualisation selon l'une quelconque des revendications précédentes, dans lequel un diamètre interne du corps est uniforme entre les première et deuxième extrémités.

15. Dispositif de visualisation selon l'une quelconque des revendications précédentes, dans lequel un diamètre interne de la première extrémité du corps est supérieur à un diamètre interne de la deuxième extrémité du corps.
